# EUROPEAN PATENT APPLICATION

(11) **EP 1 798 215 A1**
(43) Date of publication of application: **20.06.2007**
(21) Application number: 05027422.4
(22) Date of filing: 14.12.2005
(51) Int. Cl.: C07C 217/48, C07C 213/10

(54) **Polymorph of atomoxetine hydrochloride in crystalline form**

(71) Applicant: SOLMAG S.p.A., 26837 Mulazzano LO (IT)
(72) Inventor: Malpezzi, Luciana, 20131 Milano (IT); Bedeschi, Angelo, Cassino d'Alberi 26837 Mulazzano (LO) (IT); Pizzocaro, Roberta, Cassino d'Alberi 26837 Mulazzano (LO) (IT)
(74) Representative: Minoja, Fabrizio

(57) **Abstract**

The present invention discloses a process for the preparation of atomoxetine hydrochloride in a pure crystalline form, characterised by an XRPD spectrum as in Fig 1. It is also object of the present invention to provide methods to obtain pure atomoxetine hydrochloride without the use of methylene chloride.

## Description

### Field of the Invention

The present invention provides crystalline atomoxetine hydrochloride and methods for its preparation by means of safe crystallization conditions and avoiding the use of halogenated solvents.

### Background of the invention

Atomoxetine hydrochloride or (R)-N-methyl-γ-(2-methylphenoxy)-benzenepropanamine hydrochloride has been used as a medicament in the treatment of attention deficit disorders. The X-ray powder diffraction data of atomoxetine hydrochloride have been reported into ICDD database (International Centre Diffraction Data) by Ely Lilly since 1987. According to EP 0052492, crystallization of Atomoxetine is performed in methylene chloride/ethyl acetate mixtures. However, halogenated solvents are harmful, and their use is questioned due to their potential environmental impact. In addition, being the crystallization the last step in the manufacture, methylene chloride traces are likely to be retained in the final product. It is therefore important to substitute halogenated solvents with more suitable ones, from the point of view of the industrial production, environmental impact, and purity of the final product.

It has now been found that crystallization of atomoxetine can be advantageously carried out with different solvent mixtures without methylene chloride.

Crystalline form of atomoxetine, characterised by an X-ray powder diffraction pattern having a number of additional, significant peaks, that were not present in the spectrum reported in the database, have also been obtained and are a further object of the invention.

A preferred crystalline nature of the present invention is of monocrystalline form and is characterised by crystal size in the range up to millimeter.

One of said crystals was analysed by single crystal X-ray diffraction.

The study has led to the determination of the molecular and crystalline structure of atomoxetine hydrochloride. The X-ray structure clearly shows that the crystalline material is atomoxetine hydrochloride. To test the crystalline homogeneity of the product, the above sample was subjected, after accurate milling, to X-ray powder diffraction ("XRPD"). The obtained spectrum was compared with the theoretical X-ray powder diffraction spectrum, calculated from the geometrical data of the single crystal X-ray analysis. Necessarily, the calculated diffractogram contains all and exclusively only the diffraction peaks of the pure substance. The two diffractograms, the experimental one and the calculated one, are superimposable, showing the same angular sequence of the diffraction peaks and relative intensities of the corresponding peaks very similar. Some differences observed in the peak intensities can be imputed to "preferred orientation" effect, typically arising from crystalline morphology and/or analytical sample preparation.

This test shows the homogeneity of the sample and the crystalline purity of the product.

It has now been surprisingly found that the XRPD spectrum of the atomoxetine HCl obtained as here described, shows a number of additional and significant diffraction peaks with respect to the one reported in ICDD (International Centre Diffraction Data) by Ely Lilly in 1987, that appears therefore to be approximate or incomplete.

### Summary of the invention

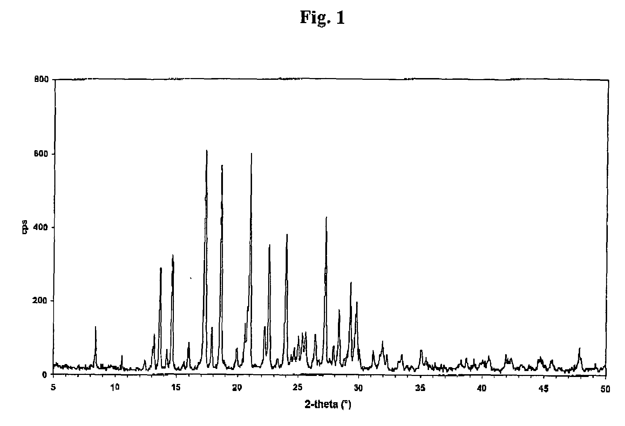

It is therefore an object of the present invention a process for the preparation of atomoxetine hydrochloride in a pure crystalline form, characterised by an XRPD spectrum as in Fig 1. It is also object of the present invention to provide methods to obtain pure atomoxetine hydrochloride without the use of methylene chloride.

In addition, the invention refers to atomoxetine hydrochloride having an X-ray powder diffraction pattern different than that reported in ICDD (International Centre Diffraction Data) by Ely Lilly in 1987.

### Brief description of the drawings

Fig.1 shows the X-ray powder diffractogram of crystalline atomoxetine hydrochloride.

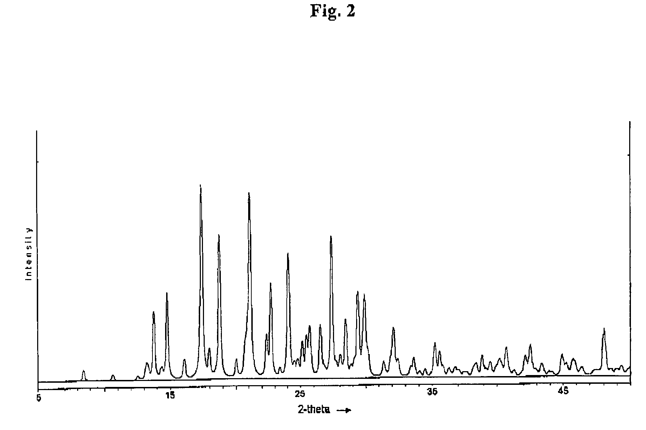

Fig. 2 shows the theoretical X-ray powder diffractogram calculated from the single crystal X-ray data.

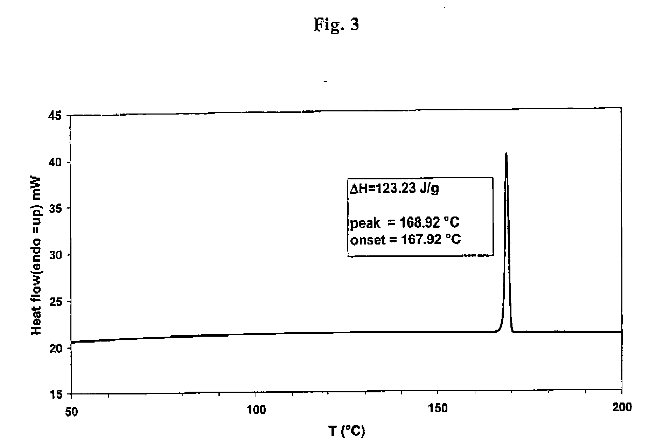

Fig. 3 shows the thermogram obtained by differential scanning calorimetry.

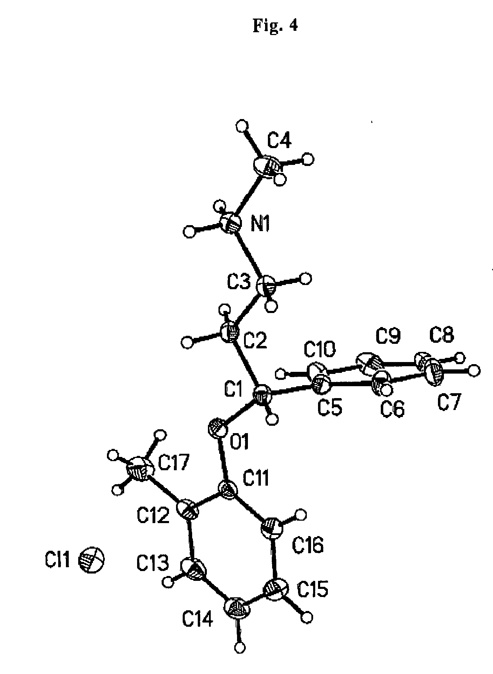

Fig. 4 shows the molecular structure of the atomoxetine hydrochloride, with the atom-numbering scheme.

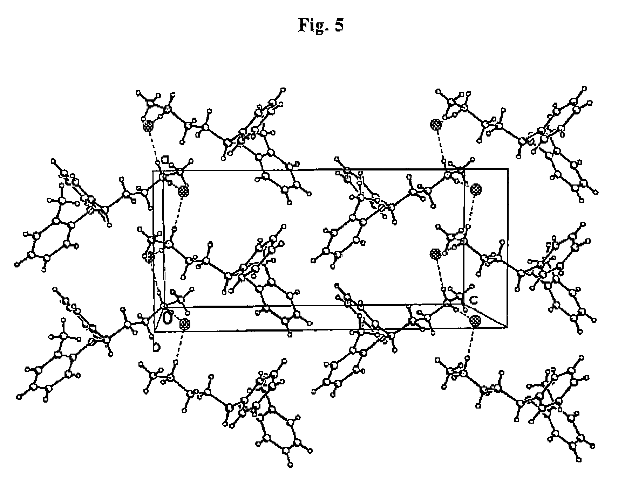

Fig. 5 shows a packing diagram of the compound, viewed along the b axis

### Detailed description of the invention

The present invention provides a crystalline form of Atomoxetine hydrochloride, at least 95% pure, obtainable without using methylene chloride as solvent.

The characterization of the crystalline pure form by X-ray powder and single crystal x-ray diffraction is reported.

Accordingly, the present invention provides atomoxetine hydrochloride crystalline form having a typical X-ray powder diffraction pattern ("XRPD") of FIG. 1. The crystalline form is also characterized by lattice spacings (d values) and relative intensities of its X-ray powder pattern as reported in table 1. The XRPD pattern has been collected using CuKα (λ = 1.5418 A) radiation, with a step width of 0.04°.

The crystalline form is also characterized by having the following crystal cell parameters: a = 7.355(1)Å, b = 13.325(1)Å, c = 16.680(2)Å, Orthorhombic System, space group P2₁2₁2₁, and the atomic coordinates of the molecule as reported in Table 2

**Table 1. List of the most prominent peaks of the X-ray powder diffraction pattern of Atomoxetine Hydroclhoride**

| **Nr. #** | **2-theta** | **d** | **Intensity** | **I/Imax** |
|---|---|---|---|---|
| | **(deg)** | **(Å)** | **(cps)** | **(%)** |
| 1 | 8,48 | 10,419 | 139 | 23 |
| 2 | 10,60 | 8,339 | 51 | 8 |
| 3 | 12,52 | 7,064 | 37 | 6 |
| 4 | 13,24 | 6,682 | 73 | 12 |
| 5 | 13,72 | 6,449 | 247 | 41 |
| 6 | 14,28 | 6,197 | 56 | 9 |
| 7 | 14,72 | 6,013 | 321 | 53 |
| 8 | 16,04 | 5,521 | 86 | 14 |
| 9 | 17,36 | 5,104 | 605 | 100 |
| 10 | 17,92 | 4,946 | 119 | 20 |
| 11 | 18,72 | 4,736 | 577 | 95 |
| 12 | 20,00 | 4,436 | 70 | 12 |
| 13 | 20,68 | 4,292 | 137 | 23 |
| 14 | 20,88 | 4,251 | 183 | 30 |
| 15 | 21,08 | 4,211 | 600 | 99 |
| 16 | 22,28 | 3,987 | 123 | 20 |
| 17 | 22,64 | 3,924 | 349 | 58 |
| 18 | 23,36 | 3,805 | 43 | 7 |
| 19 | 24,08 | 3,693 | 313 | 52 |
| 20 | 24,52 | 3,628 | 48 | 8 |
| 21 | 24,76 | 3,593 | 73 | 12 |
| 22 | 25,08 | 3,548 | 103 | 17 |
| 23 | 25,40 | 3,504 | 111 | 18 |
| 24 | 25,68 | 3,466 | 114 | 19 |
| 25 | 26,44 | 3,368 | 110 | 18 |
| 26 | 26,74 | 3,331 | 39 | 6 |
| 27 | 27,28 | 3,266 | 427 | 71 |
| 28 | 27,68 | 3,220 | 45 | 7 |
| 29 | 27,96 | 3,189 | 79 | 13 |
| 30 | 28,40 | 3,140 | 174 | 29 |
| 31 | 28,84 | 3,093 | 42 | 7 |
| 32 | 29,08 | 3,068 | 56 | 9 |
| 33 | 29,36 | 3,040 | 249 | 41 |
| 34 | 29,84 | 2,992 | 157 | 26 |
| 35 | 30,12 | 2,965 | 68 | 11 |
| 36 | 31,28 | 2,857 | 54 | 9 |
| 37 | 31,80 | 2,812 | 53 | 9 |
| 38 | 32,00 | 2,795 | 91 | 15 |
| 39 | 32,40 | 2,761 | 54 | 9 |
| 40 | 33,32 | 2,687 | 35 | 6 |
| 41 | 33,60 | 2,665 | 53 | 9 |
| 42 | 35,12 | 2,553 | 67 | 11 |
| 43 | 35,52 | 2,525 | 46 | 8 |
| 44 | 35,80 | 2,506 | 28 | 5 |
| 45 | 36,20 | 2,479 | 33 | 5 |
| 46 | 36,72 | 2,446 | 29 | 5 |
| 47 | 38,36 | 2,345 | 42 | 7 |
| 48 | 38,80 | 2,319 | 48 | 8 |
| 49 | 39,40 | 2,285 | 43 | 7 |
| 50 | 39,92 | 2,257 | 32 | 5 |
| 51 | 40,16 | 2,244 | 35 | 6 |
| 52 | 40,28 | 2,237 | 39 | 6 |
| 53 | 40,60 | 2,220 | 52 | 9 |
| 54 | 42,00 | 2,149 | 55 | 9 |
| 55 | 42,16 | 2,142 | 44 | 7 |
| 56 | 42,44 | 2,128 | 45 | 7 |
| 57 | 42,52 | 2,124 | 31 | 5 |
| 58 | 43,28 | 2,089 | 29 | 5 |
| 59 | 43,88 | 2,062 | 28 | 5 |
| 60 | 44,68 | 2,027 | 40 | 7 |
| 61 | 44,84 | 2,020 | 48 | 8 |
| 62 | 45,60 | 1,988 | 39 | 6 |
| 63 | 47,92 | 1,897 | 70 | 12 |
| 64 | 48,12 | 1,889 | 36 | 6 |
| 65 | 49,24 | 1,849 | 33 | 5 |
| 66 | 49,50 | 1,840 | 29 | 5 |

**Table 2. Fractional atomic coordinates of Atomoxetine hydroclhoride (x 10⁴), with equivalent isotropic displacement parameters (Å²)**

| **Atom** | **x** | **y** | **z** | **Ueq** |
|---|---|---|---|---|
| Cl(1) | 11583(1) | 3248(1) | 4389(1) | 54(1) |
| O(1) | 2935(3) | 2477(1) | 7552(1) | 49(1) |
| N(1) | 474(3) | 1175(2) | 5133(1) | 44(1) |
| C(1) | 2961(4) | 1538(2) | 7128(1) | 40(1) |
| C(2) | 1792(4) | 1762(2) | 6395(1) | 44(1) |
| C(3) | 1705(4) | 908(2) | 5802(1) | 42(1) |
| C(4) | 107(5) | 324(3) | 4579(2) | 62(1) |
| C(5) | 2209(4) | 702(2) | 7634(1) | 40(1) |
| C(6) | 2859(4) | -267(2) | 7559(2) | 51(1) |
| C(7) | 2139(6) | -1042(2) | 8007(2) | 68(1) |
| C(8) | 781(6) | -859(2) | 8546(2) | 70(1) |
| C(9) | 110(5) | 86(3) | 8622(2) | 68(1) |
| C(10) | 805(4) | 874(2) | 8168(2) | 54(1) |
| C(11) | 4053(4) | 2616(2) | 8205(1) | 43(1) |
| C(12) | 3667(4) | 3490(2) | 8639(1) | 49(1) |
| C(13) | 4764(5) | 3703(2) | 9292(2) | 63(1) |
| C(14) | 6169(6) | 3084(3) | 9516(2) | 70(1) |
| C(15) | 6525(5) | 2238(3) | 9078(2) | 71(1) |
| C(16) | 5471(5) | 1996(2) | 8418(2) | 58(1) |
| C(17) | 2149(6) | 4155(3) | 8394(2) | 79(1) |

The invention also provides a process for the preparation of the above described pure crystalline form of atomoxetine hydrochloride as crystalline powder, which comprises;
i) Dissolving crude Atomoxetine hydrochloride obtained by a known method in a suitable solvent, at a suitable temperature,
ii) Cooling the solution to a suitable temperature for a suitable time,
iii) Filtering the obtained suspension,
iv) Drying the collected crystals under vacuum, and
v) Optionally milling or micronizing the obtained crystals, to obtain a particle size suitable for the pharmaceutical manufacturing.

Suitable solvents for step i) include C₁-C₄ alcohols, such as methanol, ethanol, propanol, iso-propanol, n-butanol, sec-butyl alcohol or t-butanol; C₁-C₄ esters of organic C₂-C₄ acids, such as ethyl acetate, methyl acetate, ethyl propionate and the like; aliphatic or aromatic hydrocarbons, such as hexanes, petroleum ethers, heptanes, toluene, or xylenes and the like; ethers, such as tetrahydrofuran, dioxane, diethyl ether; water or mixtures thereof.

Preferred solvents are C₁-C₃ alcohols such as methanol, ethanol, or iso-propanol; C₁-C₄ esters of organic C₂-C₄ acids, such as ethyl acetate; aromatic hydrocarbons, such as toluene; ethers, like tetrahydrofuran; and water. Most preferred solvents include methanol, ethanol, iso-propanol, toluene, tetrahydrofuran, ethyl acetate and water or mixtures thereof.

Suitable temperatures for step i) range from 20°C to 200°C, preferably from 20°C to 150°C. The solvent reflux temperature is particularly preferred.

Suitable temperatures for step ii) range from -20°C to 40°C, preferably from 0°C to 30°C, and most preferred temperatures range from 0 to 25°C. Suitable time for step ii) ranges from overnight to several days, preferably from overnight to four days, and most preferably from one to three days.

Another object of the invention is a process for the preparation of pure atomoxetine hydrochloride in form of monocrystals of millimetric dimensions, which comprises:
i) Dissolving a crude Atomoxetine hydrochloride in a suitable solvent, at a suitable temperature;
ii) Letting the solution stand for a suitable time at 0-25°C;
iii) Filtering the obtained crystals;
iv) Drying under vacuum the collected crystals;

Preferred solvents for step i) include ethanol/ethyl acetate mixtures, or water. Suitable temperatures include temperatures ranging from 20°C to 200°C, preferably from 20°C to 150°C, The solvent reflux temperature is particularly preferred.

The solution is left to stand in step ii) at 0-25°C until the formation of well defined and separated crystals of the desired dimensions is obtained. This time depends on the temperature used.

In order to obtain a particle size suitable for the pharmaceutical manufacturing, the crystalline Atomoxetine hydrochloride obtained with the previous method, were optionally submitted to milling or micronizing

The composition containing said crystalline form may be in a form suitable for oral dosage as a tablet, capsule, suspension, ointment, lotion. These formulations may contain additional additives, such as sweetening or flavouring agents, coating and inert diluents, such as lactose and talc, binders and suspending agents, such as starch, hydroxyethylcellulose, hydroxypropyl cellulose and the like. Any conventional technique may be used for the preparation of pharmaceutical formulations according to the invention.

The invention is illustrated in more detail in the following examples.

### Example 1

Atomoxetine hydrochloride (7 g) was dissolved in ethanol/ethyl acetate at about 70-80°C. Part of the solvent was then distilled off until precipitation began. Additional ethyl acetate was then added maintaining the temperature at about 60°C until precipitation was complete. The suspension was then cooled at 0-5°C, and let standing overnight. The suspension was filtered and the solid washed with ethyl acetate, collected and dried under vacuum. Atomoxetine hydrochloride was obtained as white crystals.

A crystal having approximate dimension of about 0.4 x 0.5 x 0.6 mm, was selected, mounted on a glass fibre and subjected to single crystal X-ray diffraction. Unit cell parameters were determined using 74 reflections in the range 11.4 ≤ 2θ ≤ 86°. The atomoxetine hydrochloride was found to crystallise in the Orthorhombic System, space group P2₁2₁2₁, unit cell dimensions: a = 7.355(1)Å, b = 13.325(1)Å, c = 16.680(1)Å, V = 1634.6(3) Å³. Intensities data were collected on a Siemens P4 diffractometer, at room temperature, using graphite monochromated Cu-Kα radiation (λ = 1.54179Å), and θ/2θ scan technique. A total of 2866 reflections (2404 unique, Rᵢₙₜ = 0.056) were collected up to 130° in 29, with index range: -8 ≤ h ≥7, -15 ≤ k ≥ 15, -19 ≤ 1 ≥ 19.

The structure was solved by direct methods using SIR97 program (A. Altomare et al.: SIR97: A New Tool for Crystal Structure Determination and Refinement. J. Appl. Cryst. 1999, 32, 115-119) and refined by full-matrix least-squares procedure with SHELXL97 (G.M. Sheldrick. SHELXL-97. Program for the Refinement of Crystal Structures. 1997. University of Göttingen, Germany), with anisotropic temperature factors for non-H atoms. The final stage of refinement converged to R = 0.0481 for 2354 observed reflections, with I ≥ 2σ(I), and 210 refined parameters.

The (R) absolute stereochemistry of the chiral centre of the molecule was confirmed by the Flack parameter (x parameters = 0.04(2)), on the basis of 822 Friedel pairs.

### Example 2

Atomoxetine hydrochloride (3 g) was suspended in water (25 ml), and the suspension heated until dissolution. The solution was then cooled to 15-25°C, and stirred for 72 hours. The obtained suspension was then filtered. The solid was collected and dried under vacuum to yield crystalline atomoxetine hydrochloride having an XRPD spectrum as shown in fig. 1.

### Example 3

Atomoxetine hydrochloride (3 g) was suspended in toluene and the suspension heated until dissolution. The solution was then cooled to 15-25°C and stirred for 72 hours. The obtained suspension was then filtered. The solid was collected and dried under vacuum to yield atomoxetine hydrochloride with the same crystalline phase of example 2.

### Example 4

Atomoxetine hydrochloride (3 g) was suspended in isopropanol (50 ml), and the suspension was heated until dissolution. The solution was then cooled to 15-25°C and stirred for 72 hours. The obtained suspension was then filtered. The solid was collected and dried under vacuum to yield atomoxetine hydrochloride with the same crystalline phase of example 2.

### Example 5

Atomoxetine hydrochloride (3 g) was suspended in tetrahydrofuran (200 ml), and the suspension was heated until dissolution. The solution was then cooled to 15-25°C and stirred for 72 hours. The obtained suspension was then filtered. The solid was collected and dried under vacuum to yield atomoxetine hydrochloride with the same crystalline phase of example 2.

### Example 6

Atomoxetine hydrochloride (3 g) was dissolved in warm methanol (25 ml) until complete dissolution. The solvent was then allowed to evaporate until crystallization is complete. The obtained suspension was then filtered. The solid was collected and dried under vacuum to yield large crystals of atomoxetine hydrochloride, having the unit cell dimensions as in example 1.

## Claims

1. A crystalline form of atomoxetine hydrochloride, which shows an XRPD pattern as shown in figure 1.

2. The crystalline form of atomoxetine hydrochloride of claim 1 having an orthorhombic unit cell specified substantially as follows: a = 7.354(1)Å, b = 13.325 (1) Å, c = 16.680(2)Å, space group P2₁2₁2₁.

3. A crystalline form of claims 1 or 2 having a purity degree higher than 95%.

4. A process for the preparation the crystalline form of atomoxetine hydrochloride of claims 1-3, which comprises:
i) Dissolving crude Atomoxetine hydrochloride in a suitable solvent, at a suitable temperature,
ii) Cooling the solution to at a suitable temperature for a suitable time,
iii) Filtering the obtained suspension,
iv) Drying the collected crystals under vacuum, and
v) Optionally milling or micronizing the obtained crystals, to obtain a particle size suitable for the pharmaceutical manufacturing.

5. A process according to claim 4 wherein the solvent is ethanol/ethyl acetate.

6. A process according to claim 4 wherein the solvent is water.

7. A process according to claim 4 wherein the solvent is isopropanol.

8. A process according to claim 4 wherein the solvent is toluene.

9. Pharmaceutical compositions comprising as the active ingredient crystalline atomoxetine hydrochloride of claims 1-3.
